# FASCICULE DE BREVET EUROPEEN

(11) **EP 3 277 208 B1**
(45) Date de publication et mention de la délivrance du brevet: **12.12.2018**
(21) Numéro de dépôt: 16711203.6
(22) Date de dépôt: 15.03.2016
(51) Int. Cl.: A61B 17/3203, A61B 17/3211

(54) **SCALPEL**
SKALPELL
SCALPEL

(30) Priorité: 03.04.2015 FR 1500678
(43) Date de publication de la demande: 07.02.2018
(73) Titulaire: Scaljet, 38920 Crolles (FR)
(72) Inventeur: BENASSI, Pascal, 38190 Bernin (FR); MONOD, Pierre, 38700 Corenc (FR); VUILLAUME, André, 38330 Biviers (FR)
(74) Mandataire: Eidelsberg, Olivier Nathan
(86) Numéro de dépôt international: PCT/EP2016/055571
(87) Numéro de publication internationale: WO 2016/156031

(56) Documents cités:
- WO-A1-2010/148125
- WO-A2-03/096871
- GB-A- 1 445 488
- US-A1- 2009 254 075
- US-B1- 6 179 805

## Description

L'invention se rapporte aux scalpels utilisés en chirurgie pour la coupe et la dissection.

Le document WO 03/096871 A2 décrit un scalpel, comprenant un conduit, qui, d'une part, communique, notamment à une extrémité, avec une source de liquide sous pression et qui, d'autre part, aboutit dans deux orifices, ayant chacun un diamètre compris entre 0,03 mm et moins de 0,1 mm, et disposés de manière à donner deux jets de liquide convergents en un point de convergence le liquide étant sous une pression de 60 à 200 bar.

L'invention perfectionne ce scalpel par un scalpel plus précis et se prêtant mieux à la dissection.

Le scalpel suivant l'invention est du type ci-dessus caractérisé en ce que
- le point de convergence est à une distance entre 20 et 40 mm des orifices et l'entraxe entre les deux orifices est compris entre 2 et 6 mm

Le scalpel hydrique de l'invention utilise deux jets hydriques de très faible diamètre, à très haute pression et dont l'angle de convergence est également très petit. Le petit angle entre les jets permet (en contradiction directe avec ce qu'enseigne WO 03/096 871 à la page 10, lignes 10, 14 et 15), d'une part, l'obtention d'une zone de coupe à haute énergie de grande longueur de l'ordre de 30 à 40 mm située entre l'orifice des jets et leur point de convergence et, d'autre part, la création d'un éventail lamellaire liquide/air après le point de convergence. Cet éventail lamellaire est positionné à 90° par rapport au plan défini par les deux jets avant leur point de convergence. Il est donc parfaitement défini et repérable par rapport à eux.

Cet éventail liquide/air d'une longueur d'environ 20 mm possède une énergie suffisante pour disséquer les tissus sans les découper et sans traumatiser les organes tels que artères, tendons, etc.

Au-delà de la zone de l'éventail lamellaire, les jets se dispersent naturellement de par les forces de frottement dans l'air, mais il subsiste, en prolongement direct de l'éventail, une zone d'environ 20 mm, également dite de pneumatisation.

C'est cette zone qui réalise le gonflement des tissus par absorption hydrique, la dissection étant ensuite effectuée sans traumatisme par l'éventail lamellaire et la coupe ultérieure dans la première zone de l'outil en amont du point focal.

Un éventail lamellaire ne peut être obtenu qu'à la condition de limiter l'angle de convergence des jets. Celui-ci ne peut excéder 12°. Au-delà de cette valeur, il est pratiquement impossible d'obtenir un éventail lamellaire, les jets se détruisant mutuellement et perdant, de ce fait, leur énergie cinétique, rendant ainsi l'éventail lamellaire impropre à la dissection. De plus, les jets explosent sous forme de brouillard, ce qui crée une pollution visuelle et rend le scalpel impropre à tout usage. L'angle entre les deux jets se situe, de préférence, entre 3 et 7°.

Les deux paramètres, angle petit et haute pression, sont tous deux nécessaires pour pouvoir entraîner une quantité suffisante d'air situé en périphérie des jets pour former, après le point d'impact des jets, un éventail lamellaire assimilable à un scalpel et au-delà de cette zone et conserver une énergie cinétique suffisante pour atomiser le mélange hydrique/air et générer ainsi la zone dite de pneumatisation.

La haute pression est indispensable pour obtenir à la fois l'éventail lamellaire et la zone de pneumatisation, car c'est la vitesse élevée des jets qui permet d'entraîner la quantité d'air nécessaire à la formation de l'éventail.

La pression du liquide utilisé dans l'invention peut varier de 60 à 200 bar, de préférence de 80 à 150 bar. Une pression inférieure à 60 bar est insuffisante pour permettre d'effectuer aisément la combinaison coupe/dissection, tandis qu'une pression supérieure à 200 bar peut présenter un danger de coupe trop brutale.

Le diamètre petit des jets (généralement compris entre 0,07 et 0,08 mm) et la haute pression utilisée (de préférence supérieure à 80 bar) donnent une énergie cinétique élevée permettant la découpe des tissus avec un débit de liquide très faible, ce qui en favorise l'élimination par aspiration et améliore la visibilité et donc la précision de l'opération. Le diamètre réduit des jets permet, par ailleurs, d'augmenter considérablement la précision de l'opération.

Il est indispensable en chirurgie de réduire au maximum le débit de fluide, un débit trop élevé posant des problèmes quasi insolubles d'élimination dudit fluide.

Le diamètre des orifices permet à la fois de limiter le débit de fluide ainsi que les forces de frottement dans l'air, celles-ci étant proportionnelles au diamètre.

Ce diamètre est de préférence compris entre 0,07 et 0,08 mm.

L'énergie des jets de diamètre inférieur à 0,03 mm est trop faible pour pouvoir générer un effet de coupe et cela même avec une pression très élevée proche de 200 bar.

Si le diamètre des jets est supérieur ou égal à 0,1 mm, le débit hydraulique est trop grand et entraîne des difficultés d'élimination dudit liquide par aspiration.

La convergence des jets est effectuée de manière à créer un mélange du liquide constituant les jets et de l'air ambiant en formant un éventail lamellaire dont l'épaisseur est voisine du diamètre des jets. Cet éventail crée un effet de pneumatisation permettant la dissection modulable en fonction de la distance.

L'entraxe des deux orifices est, de préférence, de 3 mm environ, en allant notamment de 2 à 6 mm. L'angle entre les deux jets est inférieur à 12° et, de préférence, est compris entre 3 et 7°.

La zone de coupe est ainsi suffisamment longue pour être clairement différenciée de la zone de dissection par le chirurgien.

L'effet de coupe est obtenu en amont du point de convergence et celui de dissection en aval de ce point.

Ce scalpel peut être utilisé dans tout type de chirurgie (ouverte, laparoscopique, robot-assistée...). Sa fonction de coupe peut être opérationnelle dès l'incision cutanée, puis pour la section des plans plus profonds (aponévroses, muscles...). Sa deuxième fonction permet la dissection atraumatique des éléments nobles, comme les pédicules vasculo-nerveux et la dilacération des parenchymes.

On dirige sur l'organe à opérer un faisceau constitué de deux jets distincts convergents, qui produisent, après leur point de convergence, grâce à ladite distance permettant d'entraîner beaucoup d'air, un éventail lamellaire extrêmement fin, constitué par un mélange du liquide des jets et de l'air entraîné par les jets.

La zone en amont du point de convergence correspond à la zone de coupe du scalpel. La zone en éventail est celle utilisée pour la dissection.

Le liquide préféré constituant les jets est du sérum physiologique, qui peut être contenu dans une poche et être mis sous pression à l'aide d'une pompe.

Pour s'adapter à tout type de chirurgie, le conduit peut être monté sur une poignée, conformément aux habitudes de la chirurgie ouverte, ou bien sur un support, pour la chirurgie laparoscopique.

Il est enfin prévu un robinet d'interruption, de préférence télécommandé, de la communication entre la source et le conduit, ainsi qu'un dispositif, de préférence télécommandé, permettant de faire varier la pression des jets.

Aux dessins annexés, donnés uniquement à titre d'exemple :

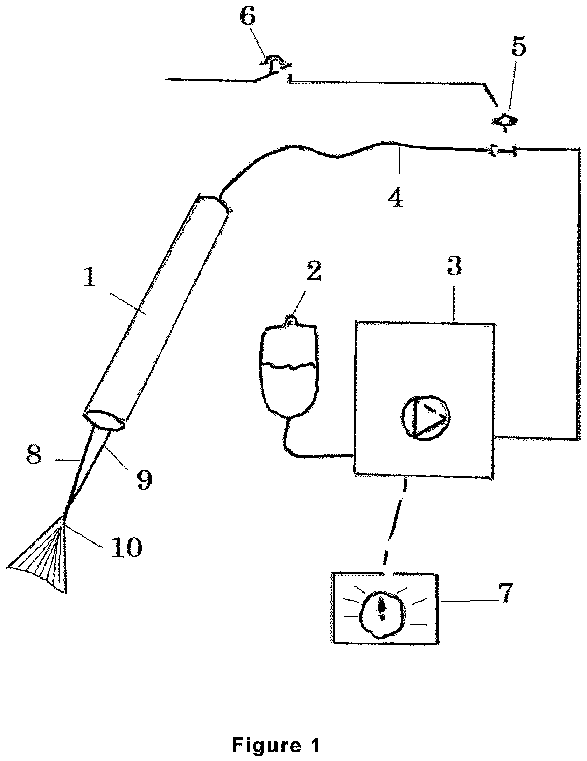

- la figure 1 représente un scalpel suivant l'invention,
- la figure 2 est une photographie du faisceau du scalpel, et
- la figure 3 est un schéma du faisceau du scalpel.

Le scalpel suivant l'invention comprend une poignée 1 tubulaire, définissant un conduit, qui communique, par l'intermédiaire d'une canalisation 4, avec une poche 2 de sérum physiologique mis sous pression par une pompe 3. Sur cette canalisation 4, est monté un robinet 5 à télécommande 6. La pompe 3 comporte un dispositif 7 permettant de faire varier la pression du sérum physiologique envoyé dans le conduit du manche 1.

Ce conduit débouche à l'extérieur, à l'extrémité opposée à celle où arrive la canalisation 4, par deux orifices, chacun d'un diamètre de 0,08 mm, disposés de manière à donner deux jets 8, 9 convergents en un point 10.

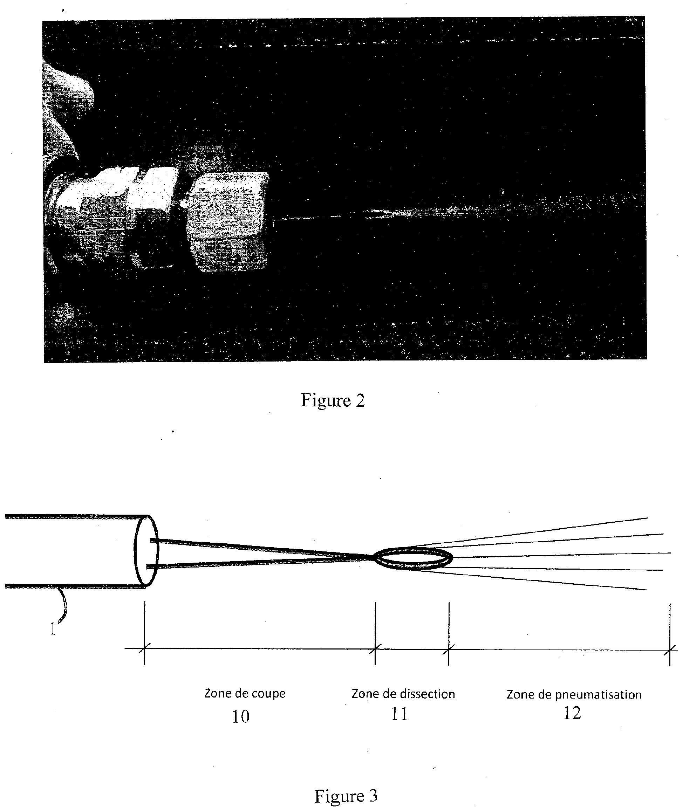

Le faisceau de sérum physiologique photographié de la figure 2 et schématisé à la figure 3 comprend, de l'amont vers l'aval, une zone 10 de coupe où les deux jets convergent l'un vers l'autre, séparée d'une zone 11 de dissection par le point de convergence. La zone 11 de dissection est suivie d'une zone 12 de pneumatisation divergente. La zone 11 de dissection a la forme d'un éventail avec écartement lamellaire.

## Revendications

1. Scalpel qui comprend un conduit (1) qui, d'une part, communique avec une source (2) de liquide sous pression et qui, d'autre part, aboutit dans deux orifices, ayant chacun un diamètre compris entre 0,03 mm et moins de 0,1 mm et disposés de manière à donner deux jets (8, 9) de liquide convergents en un point (10) de convergence, le liquide étant sous une pression de 60 à 200 bar,
**caractérisé en ce que**
- le point (10) de convergence est à une distance entre 20 et 40 mm des orifices et l'entraxe entre les deux orifices est compris entre 2 et 6 mm.

2. Scalpel suivant la revendication 1,
**caractérisé en ce que** le liquide est sous une pression de 80 à 150 bar.

3. Scalpel suivant l'une des revendications précédentes,
**caractérisé en ce que** le diamètre des orifices est compris entre 0,07 et 0,08 mm.

4. Scalpel suivant l'une des revendications précédentes,
**caractérisé en ce que** l'angle entre les deux jets est inférieur à 12°.

5. Scalpel suivant la revendication 4, **caractérisé en ce que** l'angle entre les deux jets est compris entre 3 et 7°.

6. Scalpel suivant l'une des revendications précédentes, **caractérisé en ce que** le liquide est du sérum physiologique.

7. Scalpel suivant l'une des revendications précédentes, **caractérisé en ce que** le conduit est ménagé dans une poignée (1) ou sur un support.

8. Scalpel suivant l'une des revendications précédentes, **caractérisé par** un robinet (5) d'interruption de la communication entre la source et le conduit.

9. Scalpel suivant l'une des revendications précédentes, **caractérisé par** un dispositif (6) permettant de faire varier la pression des jets.

## Patentansprüche

1. Skalpell, das ein Rohr umfasst (1), das zum einen mit einer Quelle (2) von Druckflüssigkeit in Verbindung steht und das zum anderen in zwei Öffnungen mündet, von denen jede einen Durchmesser zwischen 0,03 mm und weniger als 0,1 mm hat und die so angeordnet sind, um zwei Strahlen (8, 9) von Flüssigkeit abzugeben, die in einem Konvergenzpunkt (10) zusammenlaufen, wobei die Flüssigkeit unter einem Druck von 60 bis 200 bar steht,
**dadurch gekennzeichnet, dass**
- der Konvergenzpunkt (10) sich in einer Entfernung zwischen 20 und 40 mm von den Öffnungen befindet und der Mittenabstand zwischen den beiden Öffnungen zwischen 2 und 6 mm beträgt.

2. Skalpell nach Anspruch 1,
**dadurch gekennzeichnet, dass** die Flüssigkeit unter einem Druck von 80 bis 150 bar steht.

3. Skalpell nach einem der vorhergehenden Ansprüche,
**dadurch gekennzeichnet, dass** der Durchmesser der Öffnungen zwischen 0,07 und 0,08 mm beträgt.

4. Skalpell nach einem der vorstehenden Ansprüche,
**dadurch gekennzeichnet, dass** der Winkel zwischen den beiden Strahlen weniger als 12° beträgt.

5. Skalpell nach Anspruch 4,
**dadurch gekennzeichnet, dass** der Winkel zwischen den beiden Strahlen zwischen 3 und 7° beträgt.

6. Skalpell nach einem der vorhergehenden Ansprüche,
**dadurch gekennzeichnet, dass** die Flüssigkeit eine Kochsalzlösung ist.

7. Skalpell nach einem der vorhergehenden Ansprüche,
**dadurch gekennzeichnet, dass** das Rohr in einem Handgriff (1) oder auf einer Halterung eingerichtet ist.

8. Skalpell nach einem der vorhergehenden Ansprüche,
**gekennzeichnet durch** einen Hahn (5) zur Unterbrechung der Verbindung zwischen der Quelle und dem Rohr.

9. Skalpell nach einem der vorhergehenden Ansprüche,
**gekennzeichnet durch** eine Vorrichtung (6), die es ermöglicht, den Druck der Strahlen zu variieren.

## Claims

1. A scalpel that comprises a conduit (1) which, on the one hand, communicates with a source (2) of pressurized liquid and which, on the other hand, terminates in two openings, each having a diameter of between 0.03 mm and less than 0.1 mm, and disposed such as to supply two liquid jets (8, 9) that converge at a convergence point (10), said liquid being at a pressure of 60 to 200 bar
**characterized in that**
- the convergence point (10) is between 20 and 40 mm away from the openings, and the spacing between the two openings is between 2 and 6 mm.

2. The scalpel according to Claim 1,
**characterized in that** the liquid is at a pressure of 80 to 150 bar.

3. The scalpel according to any of the preceding claims, **characterized in that** the diameter of the openings is between 0.07 and 0.08 mm.

4. The scalpel according to any of the preceding claims, **characterized in that** the angle between the two jets is less than 12°.

5. The scalpel according to Claim 4, **characterized in that** the angle between the two jets is between 3 and 7°.

6. The scalpel according to any of the preceding claims, **characterized in that** the liquid is physiological serum.

7. The scalpel according to any of the preceding claims, **characterized in that** the conduit is arranged in a handle (1) or on a support.

8. The scalpel according to any of the preceding claims, **characterized by** a faucet (5) for blocking communication between the source and the conduit.

9. The scalpel according to any of the preceding claims, **characterized by** a device (6) that makes it possible to vary the pressure of the jets.
